# EUROPEAN PATENT APPLICATION

(11) **EP 4 703 473 A1**
(43) Date of publication of application: **04.03.2026**
(21) Application number: 24795574.3
(22) Date of filing: 27.02.2024
(51) Int. Cl.: C12N 15/117, A61K 31/711, A61P 37/02, A61P 35/00, A61P 3/00, A61P 29/00

(54) **NON-METHYLATED CPG OLIGODEOXYNUCLEOTIDE AND APPLICATION THEREOF**

(30) Priority: 25.04.2023 CN 202310456082
(71) Applicant: Beijing Ray Medicine Biotechnology Co., Ltd., Beijing 100094 (CN)
(72) Inventor: WANG, Jishu, Beijing 100094 (CN); HU, Tanyu, Beijing 100094 (CN); ZHU, Jianjun, Beijing 100094 (CN); HUANG, Tao, Beijing 100094 (CN)
(74) Representative: dompatent
(86) International application number: PCT/CN2024/078730
(87) International publication number: WO 2024/222186

(57) **Abstract**

The present invention provides a non-methylated cytosine-phosphate-guanine dinucleotide oligodeoxynucleotide and a B lymphocyte coupled thereto, the B lymphocyte can target and migrate to the diseased lymphoid organs and inhibit the B lymphocytes activated under pathological conditions, and has a targeted therapeutic effect on pathological damage caused by B lymphocyte activation.

## Description

### TECHNICAL FIELD

The disclosure discloses an oligodeoxynucleotide, belonging to the field of nucleic acid technology.

### BACKGROUND TECHNOLOGY

Toll like receptors (Toll-like receptors, TLR) are an important class of protein molecules involved in non-specific immunity (innate immunity). Among them, human Toll like receptor 9 (TLR9) is mainly expressed in B lymphocytes and CD123⁺DC cells, and activated B lymphocytes have higher expression levels than quiescent B lymphocytes. TLR9 mediates innate and adaptive immunity by recognizing pathogen-associated CpG ODNs, and is involved in various physiological and pathological processes such as inflammation, immunity, infection, and allergies. TLR9/MyD88/NF - κB is one of the important pathways in the body to defend against diseases. TLR9 agonists are used as adjuncts in vaccines to enhance immune responses, and products such as hepatitis B vaccine Heplisav and COVID19 vaccine have been approved for marketing. Meanwhile, TLR9 is currently one of the most promising targets in tumor immunotherapy, with enormous potential for monotherapy and in combination with other anti-tumor therapies. As a type of innate immune stimulatory molecule, TLR agonists can be used in combination with the checkpoint inhibitors to activate the human immune system, enhance the anti-tumor activity of checkpoint inhibitors, and strengthen immunity to refractory tumor antigens. Currently, most of them are undergoing phase I/II clinical trials.

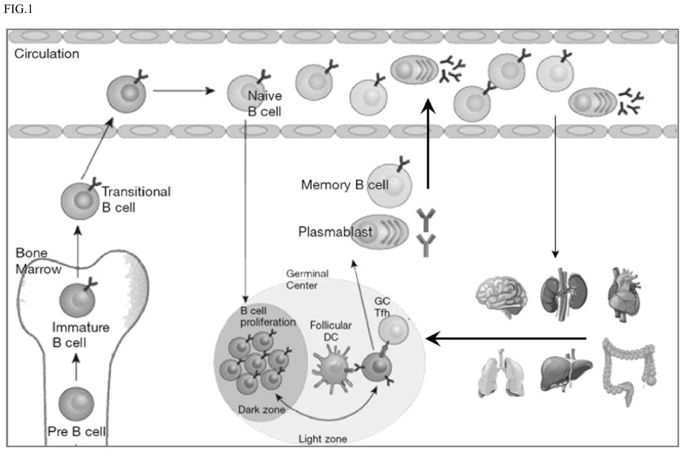

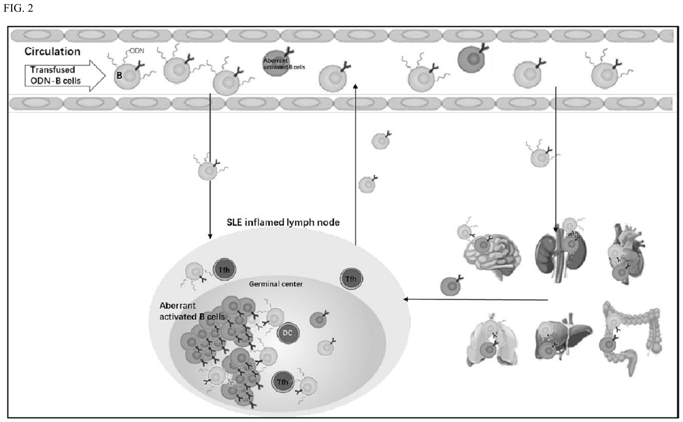

If the immune response is too strong, it can cause damage to the body (such as cellular tissue damage and functional abnormalities), leading to the emergence and development of autoimmune diseases. At present, it is believed that the mechanisms causing autoimmune tissue damage mainly include immune damage mediated by autoantibodies and caused by lymphocytes. Patients with systemic lupus erythematosus (SLE) may experience local or systemic lymph node enlargement, mainly in the neck, submandibular, and axillary lymph nodes, indicating abnormal activation of B lymphocytes and the production of antibodies against their own tissues, leading to various symptoms such as fatigue, joint pain, rash, and kidney problems. Recent reports suggest that TLR9 may play a role in the pathogenesis of SLE (JCI Insight. 2018 Mar 8; 3 (5): e96795). Under certain conditions, TLR9 can recognize self-DNA, activate TLR9 on B cells, and produce anti DNA and other autoantibodies. The excessive production of autoantibodies leads to an inflammatory cascade and large-scale immune response, ultimately resulting in organ damage in patients. In addition to secreting autoantibodies, B cells also secrete cytokines that promote inflammation and activate specific T cells, participating in attacks and damage to normal tissues in the body (Respir Res, 2007, 8 (1): 72-80) (Immunol Res. 2012 Sep; 53 (1-3): 58-77.). Further research has shown that the TLR9 signaling pathway is closely related to the onset of SLE, with TLR9 levels significantly elevated in SLE patients compared to healthy individuals and closely associated with SLE activity (Exp Ther Med. 2019 Apr; 17 (4): 3247-3254.). This discovery has prompted the development of TLR9-specific inhibitors, most of which inhibit the intracellular MyD88 signaling pathway and have not shown therapeutic effects in corresponding animal models. Due to the important role of B cells in the pathogenesis of SLE, a series of drugs targeting B cells have become a research and development hotspot, such as B cell clearance therapy targeting B cell surface markers (such as anti-CD20 antibodies), or inhibition agent on B cell activation (such as anti-BAFF antibodies), and even CAR-T therapy targeting B cells (anti-CD19 CAR-T). But these treatments can only effectively remove circulating B cells, and do not have a good inhibitory effect on B cells in lymphoid nidi (enlarged lymph nodes), resulting in very limited clinical benefits. At the same time, due to the clearance on B lymphocytes or severe inhibition on B lymphocyte function, side effects such as infections caused by humoral immunodeficiency may occur. Therefore, it is necessary to develop a drug for SLE that can effectively inhibit the pathological activation of B lymphocytes both in the circulation and lymphatic organs, while also preserving the humoral immune function of B lymphocytes. B lymphocytes have a special internal circulation pathway and homing characteristics (as shown in Figure 1). If B lymphocytes can carry a special drug targeting B lymphocytes and circulate in the body to the site of inflammation, the goal of "targeting recognition and inhibition" can be achieved. This not only effectively inhibits pathologically activated B lymphocytes, but also reduces side effects due to humoral immunity and off target caused by B cell deficiency (as shown in Figure 2).

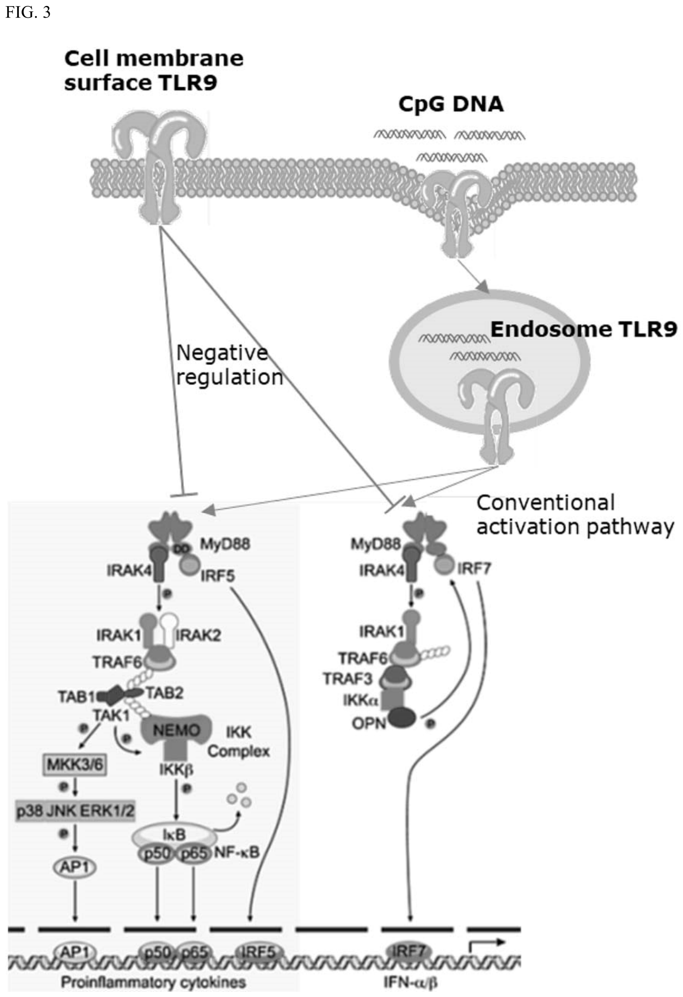

Jamin C et al.'s experiment showed that if TLR9 expressed on the B cell membrane is only activated without endocytosis and does not enter the cytoplasm, it will negatively regulate the TLR9/MyD88/NF-κB signaling pathway (J Autoimmun. 2014 Jun; 51:23-9.) (as shown in Figure 3).

TLR9 can recognize palindromic sequences whose core is unmethylated cytosine phosphate guanosine oligonucleotides (CpG oligonucleotides, CpGODN). The TLR9 receptor agonist CpG ODN can activate B cells and induce the production of various cytokines such as IL-6 and IFN - γ by binding to the TLR9 receptor. B cells in the transitional and marginal regions of lymphoid follicles are highly sensitive to TLR9 stimulation, leading to activation, proliferation, and production of immunoglobulin, participating in humoral and cellular immunity. CpG ODNs are artificially synthesized single stranded DNA short fragments containing non-methylated specific CpG dinucleotide sequences . CpG ODNs contain partial or all of the phosphorothioated backbone (phosphorothioated (PS) backbone). The structural characteristics and immune effects vary in different types of CpG ODNs, generally divided into three categories: A, B, and C. Type A of CpG ODNs have a palindromic sequence containing CpG dinucleotides as the core, poly G at both ends, and a phosphodiester backbone partially modified with thio. Through the palindromic sequence and poly G, senior structures are formed, mainly activating plasma-cell-like dendritic cells (pDC), with weak activity on B cells. Type A mainly induces a large amount of interferon I, while its induction on IL-6 is relatively weak. Type B of CpG ODN is a fully thio modified linear CpG ODN with strong immunostimulatory activity on B cells, but cannot activate plasma-cell-like dendritic cells. Type C of CpG ODN is a type of fully thio modified CpG ODN that can form dimers through palindromic sequences, possessing the activity of both type A and type B of CpG ODN. It can activate both plasma-cell-like dendritic cells and B cells. After binding to TLR9, CpG ODN enters the cytoplasm through endocytosis and binds to myeloid differentiation factor 88 (myeloid differentiation factor 88, MyD88), activating the nuclear transcription factor kappa B (nuclear factor kappa B,NF - κB) signaling system and initiating cytokine gene transcription in downstream.

Based on the principle of TLR9 specific recogniting CpGODN, the purpose of the disclosure is to seek a drug specific for B lymphocyte (such as a molecule specifically binding with TLR9), which is covalently coupled to B lymphocytes and transfused back to patients. With the special internal circulation pathway of B lymphocytes, it reaches lesions and lymphoid organs (such as enlarged lymph nodes in SLE patients), inhibits or clears pathologically activated B lymphocytes, and achieves the goal of treating diseases.

### SUMMARY OF THE DISCLOSURE

Based on the above objectives, the disclosure designs a non-methylated oligonucleotide of cytosine-phosphate-guanine dinucleotide (also referred to as "ODN" in the disclosure) based on the recognition sequence characteristics of TLR9, wherein the oligonucleotide consists of two identical the first oligodeoxynucleotide in tandem in which each the first oligodeoxynucleotide's sequence is shown as SEQ ID NO.1, or consists of the first oligodeoxynucleotide and the second oligodeoxynucleotide in tandem in which the second oligodeoxynucleotide's sequence is shown as SEQ ID NO.2. Among them, the first oligodeoxynucleotide whose sequence is shown as SEQ ID NO.1 is a type B of activated ODN (CpG-B ODNs), named as "ODN 1826", which can strongly activate B cells and TLR9-mediated NF - κB signaling. It belongs to the type B CpG ODNs of TLR9 ligand in mouse, mainly acting on B lymphocyte to stimulate the synthesis and secretion of IL-6, while its effect on the synthesis and secretion of INF - α is relatively weak.

In the disclosure, a CpG-B ODN named as "ODN 4084-F" whose sequence is SEQ ID NO.3 is also selected to serves as a control for the aforementioned ODN 1826. ODN 4084-F is a type of inhibitory ODN (Arthritis Res Ther. 2006, 8 (1): 203.), belonging to class B of inhibitory ODN, which can effectively inhibit TLR9-induced activation of lymphocyte B (Arthritis Res Ther. 2009, 11 (3): R79.), but the mechanism is not clear.

In a preferred embodiment, the two tandem oligodeoxynucleotides mentioned above are connected by two nucleosides, i.e., connected by AT or TA; Or connected by two other nucleosides, namely CG or GC; Or connect directly.

In a more preferred embodiment, the sequence of the oligonucleotide is shown as SEQ ID NO.4, named as ODNACT-1 in the disclosure, the sequence is composed of two ODN 1826 shown as SEQ ID NO.1 in tandem with AT connecting the two ODNs 1826 between them; Or the sequence of the oligonucleotide is shown as SEQ ID NO.5, named as ODNACT-2 in the disclosure, the sequence is composed of two ODN 1826 shown as SEQ ID NO.1 in tandem with GC connecting the two ODNs 1826 between them; Or the sequence of the oligonucleotide is shown as SEQ ID NO.6, named as ODNACT-3 in the disclosure, the sequence is composed of ODN 1826 shown as SEQ ID NO.1 and a type A of ODN shown as SEQ ID NO.2 in tandem directly. The 5 'and 3' ends of the above-mentioned oligodeoxynucleotides were labeled with dibenzocyclooctyne (DBCO) and biotin (Biotin), respectively.

For the above-mentioned embodiment, it is more preferred to have a diphosphate guanosine-fucose at the 3 'or 5' end of the oligodeoxynucleotide. The disclosure aims to couple the oligodeoxynucleotide to the N-GlcNac of the glycoprotein on cell membrane surface via enzymatic reaction of alpha-1,3-fucosyltransferase, and form a stable glycosidic bonds. Therefore, in order to further enzymatic reaction, the technical solution ensures that the oligodeoxynucleotide carries the donor substrate of alpha-1,3-fucosyltransferase, so that the donor substrate can undergo enzymatic reaction.

Secondly, the disclosure provides a cell coupled with the above-mentioned oligodeoxynucleotides, wherein the cell membrane has N-polysaccharide branches, and N-acetylglucosamine and/or N-acetyllactosamine are present on the N-polysaccharide branches.The α -1,3-fucosyltransferase is used in the disclosure to transfer the diphosphate guanosine fucose carried at the 3 'or 5' end of the above-mentioned oligodeoxynucleotide to GlcNAc (N-acetylglucosamine) and LacNac (N-acetyllactamine) on the N-polysaccharide branch chain through enzymatic reaction. As long as the N-GlcNac of surface glycoprotein is present on the cell membrane, the above-mentioned oligodeoxynucleotide can form stable glycosidic bonds with N-GlcNac to couple the cell to the oligodeoxynucleotide, thereby enabling the cell to exhibit various biological effects of the oligodeoxynucleotide.

In a preferred embodiment, the cells are B lymphocytes.

Thirdly, the disclosure provides the application of the prepared cells in the preparation of therapeutic drugs for tumors, autoimmune diseases, inflammatory diseases, and metabolic diseases.

As for the treatment of tumor diseases, one or more tumor-specific-recognition antigens can be conjugated to immune cells. For example, one or more tumor specific recognition antigens can be conjugated to universal CAR-T cells, such as anti-TROP2, CLDN18.2, EFGR, VEGFR, etc., to enhance the targeting of CAR-T cells, reduce off-target side effects, and improve anti-tumor efficacy.

Autoimmune diseases refer to disease states caused by the immune response of the body's immune system to its own components. The result of the body's immune response to foreign antigens is usually the clearance of the antigen. As for the immune response to body's own cells or tissues antigens, the own cells or tissues are not easily completely cleared by the effector cells of the immune system and are constantly attacked, leading to the body falling into a diseased state. TLR9 mediates innate and adaptive immunity by recognizing pathogen-associated CpG ODNs, and is involved in various physiological and pathological processes such as inflammation, immunity, infection, and allergies. TLR9/MyD88/NF-κB is one of the important pathways for the body to defend against diseases. If the immune response is too strong, it can cause damage to the body (such as cellular tissue damage and functional abnormalities), leading to the emergence and development of autoimmune diseases. At present, it is believed that the mechanisms causing autoimmune tissue damage mainly include immune damage mediated by autoantibodies and caused by lymphocytes. Lymphocytes B are abnormally activated, producing antibodies against their own tissues, leading to various symptoms such as fatigue, joint pain, rash, and kidney problems.

Therefore, the disclosure provides lymphocytes B coupled with the above-mentioned oligodeoxynucleotides. The oligodeoxynucleotides coupled to B lymphocytes can still specifically bind to TLR9 of surrounding cells, but due to being covalently coupled to the cell membrane surface, they cannot be internalized and therefore cannot activate the MyD88/NF-κB signaling pathway inside cells; Even the activated MyD88/NF-κ-B signaling pathway can be inhibited through negative regulation. The conjugated cells can be cells in the bloodstream, such as red blood cells and B lymphocyte. Due to the unique internal circulation pathway and homing characteristics of B lymphocyte, the disclosure couples ODN onto the membrane of B lymphocyte to alter the pharmacokinetic characteristics of drug distribution, metabolism, and excretion, thereby maximizing drug therapeutic properties and reducing side effects; Through the method provided by the disclosure, conjugating various therapeutic drugs, including antibodies, peptides, small molecules, and nucleic acids, onto the cell membranes of B cells, as well as stem cells and precursor cells, not only the functions of each cell are preserved completely, but also the characteristics of the conjugated drug are exhibited, which can migrate to the inflammatory site where pathologically activated B cells gather as they circulate in the body; It can achieve the goal of "targeted recognition", not only effectively inhibiting pathologically-activated B lymphocyte, but also reducing off target side effects and achieving targeted therapy. For example, coupling activating antibodies or fusion proteins of immune checkpoints, such as PD-L1 recombinant protein to the surface of B cells, utilizing the antigen-presenting effect of B cells and the inhibitory effect of PD-L1 on activity of T lymphocyte, the T lymphocyte activated by self antigens is specifically recognized and inhibited in the germinal center of lymph nodes, thereby the effectiveness of treating autoimmune diseases is improved. Therefore, the above-mentioned B lymphocyte provided by the disclosure can be applied in the preparation of therapeutic drugs for autoimmune diseases.

In a preferred embodiment, the autoimmune diseases include but are not limited to: systemic lupus erythematosus, rheumatoid arthritis, systemic vasculitis, scleroderma, dermatomyositis, mixed connective tissue disease, autoimmune hemolytic anemia, thyroid autoimmune disease, ulcerative colitis, acute glomerulonephritis, rheumatic heart disease, and diabetes.

As for inflammatory diseases, which usually include self inflammatory diseases (such as SLE mentioned above), as well as inflammatory diseases caused by other pathogenic microorganisms, such as joint (arthritis) and heart (cardioinflammation) inflammation caused by streptococcal infection in the throat. Sensitive antibacterial treatment can have a good prognosis during the acute phase. Due to incomplete treatment during the acute phase, chronic diseases are treated with strategies similar to autoimmune diseases, mainly by inhibiting the immune function of T cells, especially those around the lesion. Conjugate fusion proteins of immune checkpoints, such as PD-L1, CTLA-4, and other recombinant proteins, onto B lymphocyte, present them to T cells, and inhibit their function.

As for metabolic diseases, enzymes related to metabolism can be coupled onto the surface of blood cells, such as uric acid oxidase, to clear blood uric acid and reduce circulating uric acid levels. At the same time, acid oxidase can be carried by B cells into tissues, uric acid crystals precipitated in tissues is cleared, pain is relieved, and the goal of treating refractory gout or acute hyperuricemia nephropathy is achieved.

Finally, the disclosure provides a method for coupling the above-mentioned oligodeoxynucleotides to B lymphocyte , comprising the following steps:
(1) Modify the 3 'end of the aforementioned oligodeoxynucleotides to DBCO and the 5' end to Biotin, respectively;
(2) Add excess of GDP-Azido-fucose (GDP-Azido-Fucose, purchased from R&D Systems, item number ES101-100) to the solution of modified oligodeoxynucleotides obtained in step (1) and incubate to conjugate the two, obtaining GDP fucose-oligodeoxynucleotides;
(3) Add the GDP fucose-oligodeoxynucleotide obtained in step (2) and α-1,3-fucosyltransferase to the B lymphocyte suspension and incubate to obtain B lymphocyte conjugated with oligodeoxynucleotides. During the incubation process, GDP fucose-oligodeoxynucleotides form glycosidic bonds with GlcNac receptor molecules of surface glycoproteins on target cell membrane, thereby obtaining the B lymphocyte labeled with ODN.

In a preferred embodiment, in step (3), 100 µg/mL of α -1,3-fucosyltransferase and 10 µ M GDP fucose-oligodeoxynucleotide prepared in step (2) are added to 5 × 10⁸/mL B cells, respectively.

The oligodeoxynucleotide of non-methylated cytosine-phosphate-guanine dinucleotide designed in this invention is coupled onto the surface of B lymphocyte, without affecting the function of B lymphocyte themselves. Therefore, it ensures the B lymphocyte's unique characteristics of in vivo circulation and homing, and ensure B lymphocyte site-specifically migrate to diseased lymphoid organs such as lymph nodes followed with the circulation, achieving the goal of "targeted recognition and inhibition". It not only effectively inhibits pathologically activated lymphocyte B , but also reduces the side effects of humoral immunity and off target caused by B cell deficiency, thereby improving the therapeutic effect.

### BRIEF DESCRIPTION OF THE DRAWINGS

Figure 1. Schematic diagram of the internal circulation pathway of B lymphocyte;
Figure 2. Schematic diagram of treating SLE with local or systemic lymphadenopathy by B lymphocyte labeled with ODN;
Figure 3. Schematic diagram showing that TLR9 on the surface of cell membrane and on the endosome/lysosome membrane have opposite functions on the MyD88/NF-κB signaling pathway;
Figure 4. The TLR9-activating ODN 1826 of can stimulate the activation of mouse B cells and increase the secretion levels of mIL-6 and mTNF-a;
Figure 5. ODN-ACT1, ODN-ACT2, and ODN-ACT3 can significantly increase the secretion levels of mIL-6 and mINF-a under the same conditions;
Figure 6. ODN 4084-F can inhibit Naive B cells from being activated by ODN 1826;
Figure 7. ODN 4084-F cannot inhibit the activated B cells;
Figure 8. ODN 1826 is labeled onto the surface of the cell membrane;
Figure 9. The effect of ODN 1826 labelled on the cell membrane surface is not affected by the position of Biotin and the bond of PS, PO;
Figure 10. B cells labeled with ODN 1826 still retain their function as B cells;
Figure 11. B cells marked with ODN 1826 cannot activate each other;
Figure 12. Experimental schematic diagram of the inhibitory effect of ODNACT-2 B cells on activated B cells;
Figure 13. B cells labeled with ODNACT-2 can effectively inhibit activated B cells.

### SPECIFIC EMBODIMENTS

The present disclosure is further described with specific examples below, and the advantages and features of the present disclosure will become clearer with the description. However, these examples are only exemplary, and do not constitute any further limitations to the scope defined by the claims of the present disclosure.

### Example 1. Activation of Mouse B Cells by TLR9-activating ODN 1826

The oligonucleotides in Table 1 were synthesized by Suzhou Junji Biotechnology Co., Ltd. or Suzhou Hongxun Biotechnology Co., Ltd. according to the sequences SEQ ID NO. 1 to SEQ ID NO. 6. Among them, SEQ ID NO. 1 is based on ODN 1826. For convenient detection, dibenzocyclooctyne (DBCO) and biotin (Biotin) were labeled at the 5 'and 3' ends, respectively. To prevent degradation by nucleases, the phosphodiester bonds between the nucleotides at the ends of head and tail of ODN 1826-1 and ODN 1826-2 sequences are modified to thiophosphate bonds.

**Table 1. Oligonucleotide sequences and labels used in the disclosure**

| | ODN | | | | |
|---|---|---|---|---|---|
| SEQ ID.NO. | name | backb one | 5' | 3' | |
| 1 | ODN1826-1 | PS | Biotin | DBCO | T*C*C*ATGACGTTCCTGAC*G*T *T |
| | ODN1826-2 | PS | DBCO | Biotin | |
| | ODN1826-3 | PO | DBCO | Biotin | TCCATGACGTTCCTGACGTT |
| 2 | ODN A | PO | / | / | GGGGGGTCAACGTTGAGGGG |
| 3 | ODN4084F-1 | PS | / | / | *C*C*T*G*G*A*T*G*G*G*A*A* |
| 4 | ODN-ACT-1 | PO | DBCO | Biotin | TCCATGACGTTCCTGACGTTATT CCATGACGTTCCTGACGTT |
| 5 | ODN-ACT-2 | PO | DBCO | Biotin | TCCATGACGTTCCTGACGTTCG TCCATGACGTTCCTGACGTT |
| 6 | ODN-ACT-3 | PO | DBCO | Biotin | TCCATGACGTTCCTGACGTTGG GGGGTCAACGTTGAGGGG |

The experimental process of ODN activating lymphocyte B in mice is briefly described as follows.

### 1. Preparation of B cell suspension

Mouse B lymphocytes were taken from fresh spleens of mice ( C57BL/6J). After grinding by frosted glass plate, they were resuspended in PBS (Solarbio, P1020) and collected in a 15 mL centrifuge tube. After centrifugation for 5 minutes at 500 g to remove PBS, each spleen was resuspended in 5 mL of RBC Lysis Buffer (Solarbio, R1010) and red blood cells were lysed at room temperature for 5 minutes. After the lysis is complete, add 10 mL of PBS to terminate, mix thoroughly, and centrifuge at 500 g for 5 minutes to remove the red blood cell lysate. Wash with PBS three times, 10 mL each time. After the third resuspension, the cell suspension was filtered using a 70 µm sieve (NEST, 258368) to remove large pieces of spleen tissue. Take a small amount of cell suspension for counting (DiMai, model DF55 Vet). After centrifugation, a single spleen cell suspension was resuspended in RPMI1640 medium (Gibco, 61870-036)+10% FBS (Sigma, F8687-100mL) and adjusted to a concentration of 10⁶/mL for subsequent experiments. To obtain higher purity B cells, prepare a 10⁶/mL single spleen cell suspension according to the previous step. Take 10 mL and add it to a 10 cm culture dish (Corning, 430167). Add LPS (Solarbio, L8880) with a final concentration of 20 µg/mL and culture at 37 °C and 5% CO₂ for 48 hours. After the cultivation is completed, a small number of cells are taken and stained with CD19-FITC (Elabscience, E-AB-F0986UC, diluted 1:500). Flow cytometry (Beijing Cenglang Biotechnology Co., Ltd, model MateCyte) is used to detect the positive rate of CD19. If the positive rate is over 95%, it can be considered that the preparation of B cells is successful. Adjust the concentration of B cells to 10⁶/mL using 1640 complete culture medium, which can be used for subsequent experiments.

### 2. Activation of Mouse B Cells by ODN 1826

Spread the single cells prepared in the above steps onto a 96 well plate (Corning, 3799), with 200µL of cell suspension per well. Add corresponding concentrations of ODN1826-1, 2, and 3 (Jun Ji, S9622101703026), and incubate at 37 °C and 5% CO2 for 24 hours. After cultivation, the supernatant was collected by centrifugation and ELISA was used to detect mIL-6 (Solarbio, SEKM-0007) and mTNF - α (Solarbio, SEKM-0034). Conduct according to the manual of the kit, and read the dual wavelength of OD450 nm and OD630 nm using a microplate reader (Molecular Devices, model spetramax paradigm).

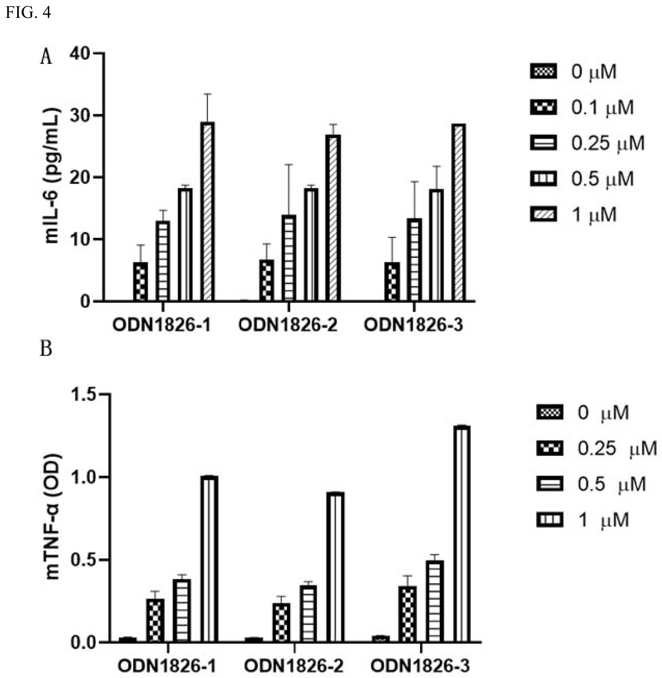

Figure 4 shows that compared with the control without ODN1826, adding ODN1826 can stimulate spleen cells to secrete IL-6 (A of Figure 4) and TNF - α (B of Figure 4), and the secretion of IL-6 and TNF - α increases with the increase of ODN1826 concentration.

As shown in Figure 4, all three oligonucleotides are based on ODN1826 and belong to class B of TLR9 agonists. Although there are some different modifications among the three types of oligonucleotides, there is no significant difference in their activation effect on B lymphocytes. As the concentration of oligonucleotides increases, the secretion of IL-6 and TNF - α increases. Therefore, subsequent TLR9-activating oligonucleotides were labeled with DBCO at the 5'end and Biotin at the 3' end, and phosphodiester bonds were used as the bonds between nucleotides.

### Example 2. Activation of mouse B cells by ODN-ACT1, ODN-ACT2, and ODN-ACT3

The activation effect of ODN 1826 as a TLR9 agonist was not ideal in Example 1. When the concentration of ODN 1826 was doubled, the secretion of mIL-6 only increased by 30%. We speculate that activation can be enhanced by concatenating ODN 1826. SEQ ID NO.4, SEQ ID NO. 5 and SEQ ID NO. 6 is named as ODNACT-1, ODNACT-2, and ODNACT-3 respectively. ODN ACT-1 connected two class B of ODNs by AT (adenine thymidine); ODN ACT-2 connects two class Bof ODNs by CG (cytosine guanine); ODN ACT-3 directly connects Class B of ODN and Class A of ODN.

The B lymphocytes of mice is from high-purity B lymphocytes that have been amplified in vitro. The experimental process is as described in Example 1.

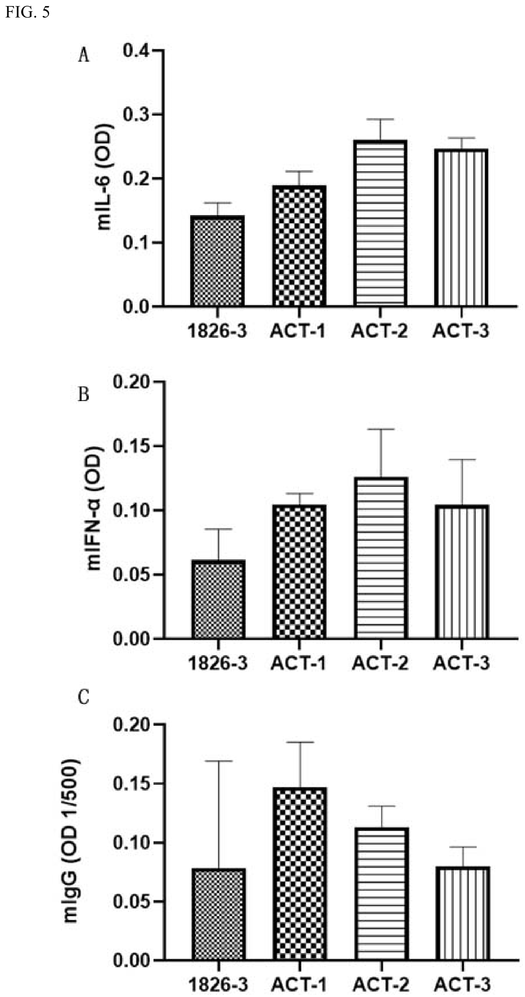

As shown in Figure 5, ODN ACT-1, ODN ACT-2 and ODNACT-3 all exhibited stronger B cell activation effects than ODN 1862-3 under the same concentration and same amount of B cells. In Figure 5, A represents IL-6 detection, B represents INF - α detection, and C represents IgG detection, but ODN ACT-1, ODN ACT-2 and ODNACT-3 of 3 kinds of TLR9 activators have different effects on B cell activation. Being class B of activator, ODN ACT-2 exhibits a stronger stimulating effect on IL-6 secretion, which is the strongest activity among the four ODN activators. This result is in line with expectations. Both ODN ACT-1 and ODN ACT-2 are ODNs connected in series by two class B of ODN, but the CpG is selected as the connecting part of ODN ACT-2, which has stronger binding affinity toTLR9. Surprisingly, ODN ACT-2 also exhibited the strongest INF - α activation effect. A class B of ODN and a class A of ODN are connected in series in ODNACT-3. It is reported in literature that the class A of ODN has a more significant activation effect on IFN - α compared to the class B of ODN, but experiments have shown that ODN ACT-2 still has a stronger stimulating effect on IFN - α secretion than ODNACT-3. In summary, the ODN ACT-2 showed a more significant stimulating effect on B cells.

### Example 3. Activation Experiment of B Lymphocytes by ODN 4084-F

ODN 4084-F is a novel inhibitory ODN composed of two nucleotide triplets, a proximal CCT and a distal GGG, separated by four nucleotides from each other. ODN 4084-F is the shortest active - inhibitory ODN, whose sequence shown as SEQ ID NO. 3. It belongs to class B of inhibitory ODN and can effectively inhibit TLR9-mediated activation of B cells (Arthritis Res Ther. 2009, 11(3):R79.) . According to literature, the inhibitory effect of ODN 4084-F depends on the thiophosphate bond, and we have also confirmed this phenomenon. In the synthesis of ODN 4084-F oligonucleotides, all thiophosphate bonds were selected instead of phosphodiester bonds.

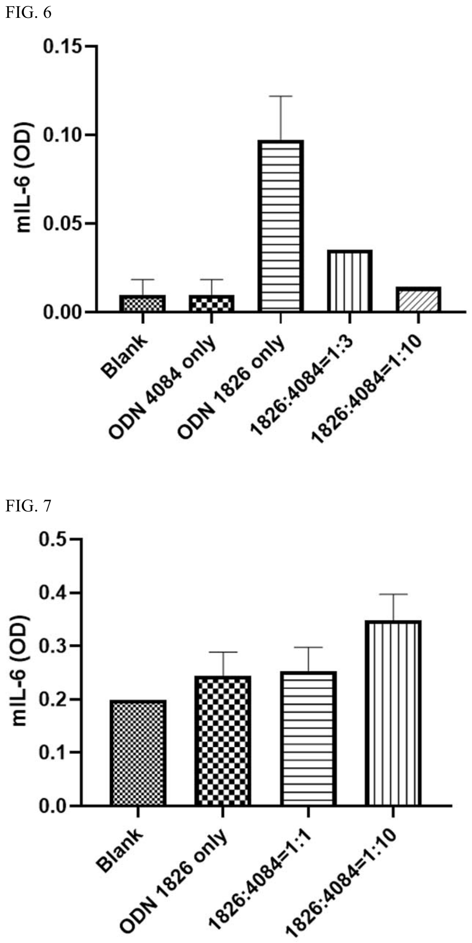

As shown in Figure 6, ODN 4084-F did not stimulate mIL-6 production in mouse B lymphocytes, while ODN 1826 was able to effectively activate B lymphocytes. When B lymphocytes were co-cultured after ODN 4084-F and ODN 1826 were mixed in a certain ratio, the production of mIL-6 mediated by ODN 1826 gradually decreased as the concentration of ODN 4084-F gradually increased, consistent with the published literature.

As shown in Figure 7, if B lymphocytes are first activated by LPS and/or mIL-4 (similar to B lymphocytes pathologically activated in SLE), the activated B cells can still be activated by ODN 1826 furtherly, but the addition of ODN 4084-F does not inhibit mIL-6 production. Therefore, ODN 4084-F can prevent Naive B cells from being activated by ODN 1826, but ODN 4084-F has no significant inhibitory effect on B cells that have already been activated. Therefore, ODN 4084-F is not suitable as a therapeutic drug, especially for SLE in the episodic phase, which may be one of the reasons why ODN 4084-F has not been used as a TLR9 inhibitor in clinical treatment.

The test process is briefly described as follows:
10⁶/mL B cells were prepared as described in Example 1, the cells were plated onto a 96-well plate (Corning, 3799), 200 µL of cell suspension per well, and a final concentration of 1 µM of ODN1826-3 (Junji, S9622101703026) or/and 1 µM, 3 µM, or 10 µM of ODN4084-F (Suzhou Hongxun, CN32260) were added , and incubated at 37 °C with 5% CO₂ for 24 h. After the culture, the supernatant was collected by centrifugation, and mIL-6 was detected by ELISA (Solarbio, SEKM-0007).

To obtain activated B cells, LPS (Solarbio, L8880) at a final concentration of 20 µg/mL and mIL-4 (GenScript, Z02996) at a final concentration of 2.5 ng/mL were added to the prepared B cells at 37 °C and 5% CO₂ for 24 h activating . Spread activated B cells onto a 96 well plate (Corning, 3799), with 200 µ L of cell suspension per well. Add ODN1826-3 (Jun Ji, S9622101703026) at a final concentration of 1 µ M and ODN4084-F (Suzhou Hongxun, CN32260) at 1 µ M or 10 µ M, and culture at 37 °C and 5% CO₂ for 24 hours. After the culture is finished, the supernatant is collected by centrifugation and mIL-6 is detected by ELISA (Solarbio, SEKM-0007) .

### Example 4. labelling B lymphocytes with oligonucleotide chains

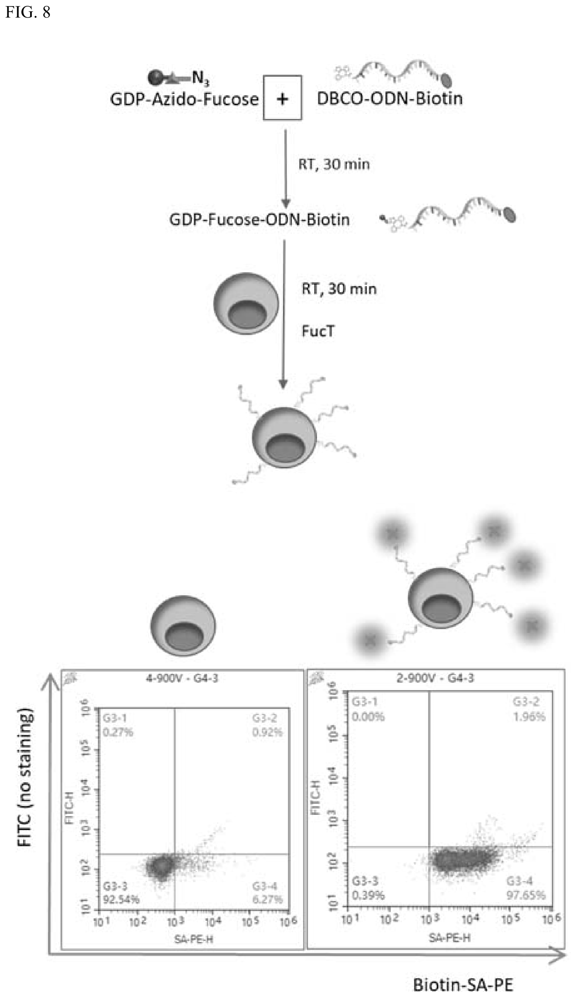

The α-1,3-fucosyltransferase (Fucosyltransferase, FucT) of Helicobacter pylori can transfer fucose from a derivative of guanosine diphosphate fucose (GDP Fucose) to GlcNAc (N-acetylglucosamine) and LacNac (N-acetylglucosamine) on the N-polysaccharide chain. By utilizing this characteristic, ODNs with GDP Fucose at the 3 'or 5' end can be coupled to N-GlcNac of surface glycoproteins on cell membrane under the action of FucT, forming stable glycosidic bonds. To achieve the above objectives, firstly, during the synthesis of oligonucleotide chains, the 3 'and 5' ends were modified with DBCO and Biotin, respectively (see Table 1). Add excess of GTP Azido fucose (GDP-Azido-Fucose) (purchased from R&D Systems, item number ES101-100) to ODN, incubate at room temperature for 30 minutes, add to B lymphocyte suspension while FucT is added too , and continue incubation for another 30 minutes. After three rounds of cell cleaning, the ODN on the cell membrane surface was detected using PE-labeled streptavidin PE (Streptavidin-PE, SA-PE), as shown in Figure 8. The specific process is described as follows.
1. The preparation of α-1,3-fucosyltransferase mutant of Helicobacter pylori is briefly described as follows:
   (1) Suzhou Junji Biotechnology Co., Ltd. was commissioned to synthesize plasmid DNA for expressing α-1,3-fucosyltransferase of Helicobacter pylori (strain ATCC 700392 / 26695) , and clone it into the vector pET41a (MilliporeSigma ^{™} pET-41a(+) DNA Vector, Catalog Number: 70-556-3). Take 2 microliters of plasmid and add 100 microliters of BL21 (DE3) competent cells (ThermoFisher, catalog number: EC0114) , mix immediately and let it sit on ice for 30 minutes.
   (2) Heat shock at 42 °C for 90 seconds and quickly ice bath for 2 minutes.
   (3) Add 500 uL LB medium, incubate at 37 °C, rpm<=200, and shake for 60 minutes.
   (4) Centrifuge at 6000rpm for 1 minute, discard most of the supernatant, leave about 100-150µL, resuspend the bacterial cells, coat them on LB plates containing Amp, and incubate overnight at 37 °C.
   (5) Small expression: Select one monoclonal and culture it in 1 mL of Amp resistant LB medium at 37 °C and 220 rpm shaking for about 5 hours. Add 2.5 mL of LB liquid medium containing Amp to the previous tube and culture it overnight at 37 °C and 220 rpm shaking.
   (6) Transfer the overnight cultured bacterial solution to 20mL LB medium containing Amp at a ratio of 1:50, at 37 °C and 220rpm, and culture until OD600=0.6 (about 3h). Add a final concentration of 0.5mM IPTG and culture at 30 °C and 220rpm for 6h.
   (7) Measure the OD600 of the culture medium, take 10OD bacterial solution, centrifuge at 10000rpm for 2 minutes, and remove the supernatant.
   (8) Resuspend the bacterial cells in 1 mL of lysis buffer (10 mM Tris HCl, pH 8.0) and place them on ice for ultrasonic lysis of cells. Ultrasonic conditions: 130W, 4 minutes on 3s, off 3s.
   (9) After the ultrasound is completed, the lysis buffer rotates at 12000rpm, Centrifuge for 10 minutes to obtain the supernatant (lysis supernatant); Centrifuge the supernatant at a speed of 125000g at 4 ° C.
   (10) According to the user manual, apply the supernatant to the HiTrap chelating HP column and elute with 20mM imidazole solution. The eluant was collected and the solvent was replaced with 50 mM Tris buffer solution (pH 8.0) through dialysis, and then further purified by gel filtration chromatography (superdex200, GE Healthcare) to obtain a protein with high purity, producing a protein with more than 98% homogeneity.
   (11) Using bovine serum albumin as the standard, the protein concentration was determined using the Bio Rad protein analysis kit based on the Bradford method.
2. Preparation of GDP-Fuchose-ODN
   100 µM GDP-Azido-Fucose (purchased from R&D Systems, item number ES101-100) and 100 µ M ODN were mixed uniformly in a ratio of 1:2 and incubated at room temperature for 30 minutes to obtain a theoretical concentration of 33 µM GDP-Fucose-ODN.
3. GDP-Fuchose-ODN coupled to cell membrane

After washing B cells with PBS, thoroughly remove the culture medium for later use. Add 100 µ g/mL FucT and 10 µM of the previously prepared GDP-Fucose-ODN to 5 × 10⁸/mL B cells, and incubate at room temperature for 30 minutes. Wash with PBS, centrifuge 500g for 5 minutes, and remove the supernatant. B cells were resuspended in PBS and stained with Streptavidin PE (BioLegend, Inc., product number 405204, diluted 1:200). They were co-incubated at 4 ° C for 30 minutes, washed once with PBS, and detected using a flow cytometer (MateCyte, product model from Beijing cenglang Biotechnology Co., Ltd.) and analyzed using NovoExpress software.

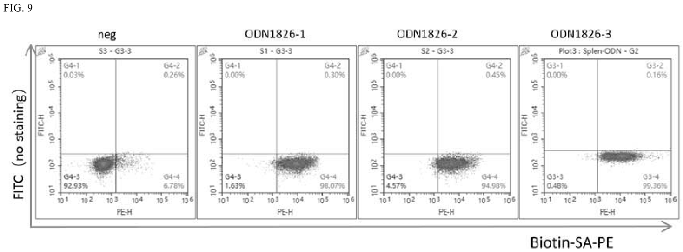

As shown in Figure 8, ODN can be quickly labeled on the surface of B lymphocyte membrane at room temperature. To compare the positions of DBCO and Biotin, as well as the effects of PS and PO bonds on labeling efficiency, B lymphocytes were labeled with ODN 1826-1, ODN 1826-2, and ODN 1826-3 under the same conditions. As shown in Figure 9, ODN 1826-1, ODN 1826-2, and ODN 1826-3 can effectively be labelled on the surface of B lymphocytes, while the positions of DBCO and Biotin, as well as PS and PO bonds, have no significant influence on the labeling of mouse B lymphocytes.

### Example 5. Experiment for confirmation of B cell function being preserved after labeled with oligonucleotide chains

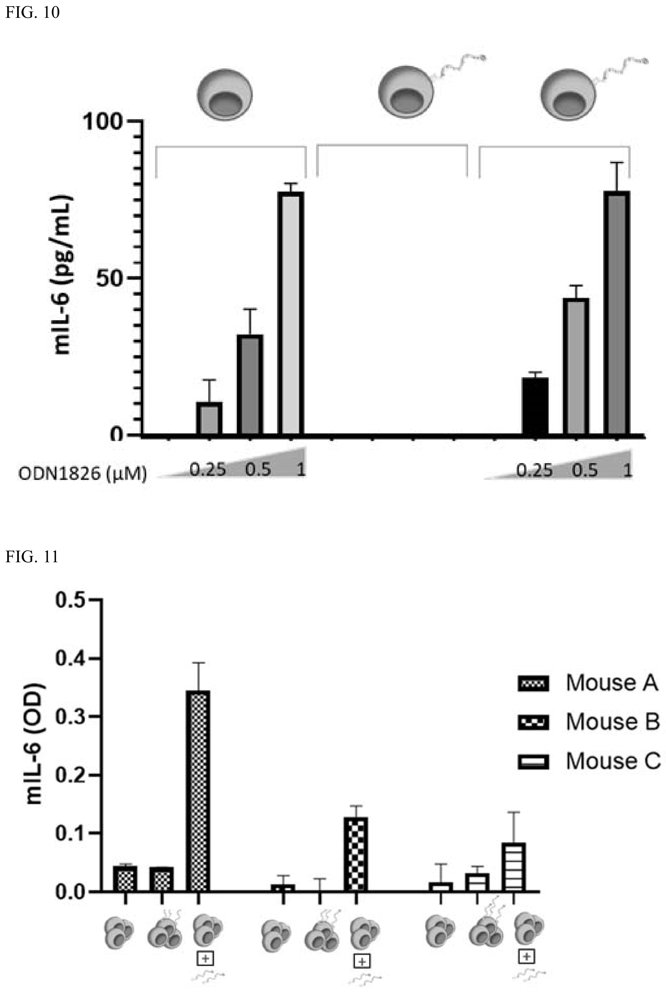

Lymphocyte homing is one of the characteristics of B lymphocytes, which is a directional movement that includes movement from peripheral tissues to central lymphoid organs, as well as movement from central lymphoid organs to peripheral lymphoid organs, lymphocyte recycling, and migration of lymphocytes to inflammatory sites( such as skin, intestinal mucosa, and joint synovium). The essence of the homing process is the interaction between receptors on the surface of lymphocyte membranes and adhesion molecules of vascular endothelial cells in various tissues and organs. During the preparation of ODN-labeled lymphocytes, B lymphocytes were not subjected to chemical or physical treatment, and had no effect on the amino acids on the cell membrane surface. To confirm that ODN-labeled B lymphocytes still have complete membrane function, we compared the response of B lymphocytes to LPS, IL-4, and TLR9 activators before and after ODN labeling. As shown in Figure 10, B lymphocytes labeled with ODN showed a good response to TLR9 activators in a dose-dependent manner, same as before labeling. Therefore, the labeling method provided by the disclosure is non-invasive for B lymphocytes and the process is mild. This method ensures the reinfusion of B lymphocytes carrying ODN into the body still retaining the characteristics of B lymphocytes. With their unique homing and migration pathways, they are located in the diseased B lymphocyte tissue and exert their pharmacological effects.
1 . The experimental process of Figure 10 is briefly described as follows:
   (1) Prepare 10⁶/mL B cells using the method described in Example 1 for future use.
   (2) Prepare B cells coupled with GDP-Focus-ODN using the method described in Example 4. After confirming the success d by flow cytometry detection, resuspend at 1E6/mL.
   (3) Spread the prepared cells onto a 96 well plate (Corning, 3799), with 200µL of cell suspension per well. Add ODN1826-3 (Jun Ji, S9622101703026) at final concentrations of 0.25µM, 0.5µM, and 1µM, and incubate at 37 °C and 5% CO₂ for 24 hours. After cultivation, collect the supernatant by centrifugation and detect mIL-6 by ELISA (Solarbio, SEKM-0007).

When B cells labeled with ODN 1826 are cultured, can cells recognize TLR9 on each other's cell membrane through ODN 1826 on their respective plasma membranes, thereby activating each other? To answer this question, we extracted B lymphocytes from three mice separately, and some of them were labeled with ODN 1826 on the cell membrane surface according to the method in Example 4. Naïve B lymphocytes and ODN-labeled B lymphocytes were cultured separately for 24 hours, and the supernatant was taken to detect the level of mIL-6. As a positive control signal for the activated B lymphocytes, a portion of Naive B lymphocytes were cultured with free ODN 1826. As shown in Figure 11, when B cells labeled with ODN 1826 were cultured, the level of mIL-6 in the culture supernatant did not increase, indicating that cells did not activate each other due to the labeling of ODN 1826 on the cell membrane and maintained a similar state to Naive B cells.

2. The experimental process of Figure 11 is briefly described as follows:
(1) Take fresh spleens from 3 mice and prepare 10⁶/mL B cells using the method described in Example 1 for future use.
(2) Prepare B cells coupled with GDP-Fucose-ODN using the method described in Example 4, and resuspend at 10⁶/mL after confirming the success by flow cytometry detection.
(3) Spread the prepared cells onto a 96 well plate (Corning, 3799), with 200µL of cell suspension per well. Add ODN1826-3 (Jun Ji, S9622101703026) at final concentrations of 1 µM, and incubate at 37 °C and 5% CO₂ for 24 hours. After cultivation, collect the supernatant by centrifugation and detect mIL-6 by ELISA(Solarbio, SEKM-0007).

### Example 6. Application of ODNACT-2 B cells to activated B cells

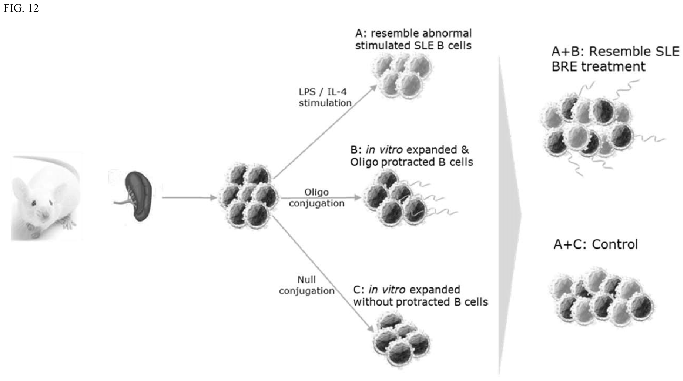

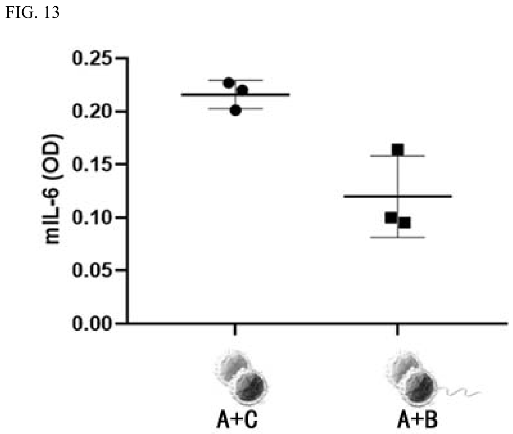

As described in Example 5, B cells labeled with ODN 1826 still exhibit the characteristics of Naive B cells and do not activate each other due to ODN. Therefore, if ODN 1826 labeled on the cell membrane can bind and activate TLR9, but does not induce TLR9/ODN internalization, negative regulation on MyD88 will occur. This also makes it feasible as an immunosuppressive drug. ODNACT-2 exhibits a stronger ability to bind TLR9 than ODN 1826. To confirm the inhibitory effect of ODNACT-2-labeled B cells on activated B lymphocytes, we grouped B cells from the same mouse, as shown in Figure 12. Group A was stimulated with LPS and mIL-4 to simulate activated B lymphocyte cells from SLE patients; Group B underwent ODNACT-2 coupled and labelled; Group C serves as the control, consisting of NaiveB lymphocytes. The cells of Group A were mixed respectively with Group B and Group C for culture, and after 24 hours, the level of mIL-6 in the culture supernatant was measured (0.21 ng/mL ± 0.013 ng/mL for Group A+C; 0.11 ng/mL ± 0.038 ng/mL for A+B group). As shown in Figure 13, ODNACT-2 labeled on the cell membrane surface can effectively inhibit activated B cells.

The experimental process is briefly described as follows:
1. Obtain activated B cells (i.e. group A in the figure) using the method described in Example 3, and spread the activated B cells onto a 96 well plate (Corning, 3799) with 100 µL of cell suspension per well.
2. Prepare 10⁶/mL B cells using the method described in Example 1, i.e. Group C in the figure.
3. Prepare B cells coupled with GDP-Fucose-ODN according to the method described in Example 4. After confirming the success by flow cytometry detection, resuspend at 10⁶/mL, which is Group B in the figure.
4. The prepared cells of group C and group B were added to the wells spread with group A cells at a rate of 100µL per well, and cultured at 37 °C and 5% CO₂ for 24 hours. After cultivation, collect the supernatant by centrifugation and detect mIL-6 by ELISA (Solarbio, SEKM-0007) .

In summary, the disclosure designs and synthesizes a highly active ODN and provides a method for coupling onto the surface of B lymphocytes. The provided method can rapidly couple OND onto the surface of B lymphocyte membrane under mild conditions without affecting the function of the cell itself. Therefore, it ensures the unique characteristics of internal circulation and homing of B lymphocytes, which can selectively migrate to diseased lymphoid organs such as lymph nodes, thereby inhibiting the activation of B lymphocytes under pathological conditions, achieving therapeutic goals, improving tissue targeting, and enhancing drug efficacy.

## Claims

1. A non-methylated oligonucleotide of cytosine-phosphate-guanine dinucleotide, wherein the oligonucleotide consists of two identical the first oligodeoxynucleotides in tandem in which each the first oligodeoxynucleotide's sequence is shown as SEQ ID NO.1, or consists of the first oligodeoxynucleotide and the second oligodeoxynucleotide in tandem in which the second oligodeoxynucleotide's sequence is shown as SEQ ID NO.2.

2. The non-methylated oligonucleotide of cytosine-phosphate-guanine dinucleotide of claim 1, wherein the oligodeoxynucleotides in tandem are connected by a adenine and a thymine, or connected by a cytosine and a guanine, or connected directly.

3. The non-methylated oligonucleotide of cytosine-phosphate-guanine dinucleotide of claim 2, wherein the sequence of the oligonucleotide is shown as SEQ ID NO.4, SEQ ID NO.5 or SEQ ID NO.6.

4. The non-methylated oligonucleotide of cytosine-phosphate-guanine dinucleotide of any one of claim 1-3, wherein there is a diphosphate guanosine-fucose at the 3 'or 5' end of the oligodeoxynucleotide.

5. A cell coupled with the non-methylated oligonucleotide of cytosine-phosphate-guanine dinucleotide of claim 4, wherein the cell membrane has N-polysaccharide branches, and N-acetylglucosamine and/or N-acetyllactosamine are present on the N-polysaccharide branches.

6. The cell of claim 5, wherein the cell is B lymphocyte or T lymphocyte.

7. The application of the cell of claim 6 in the preparation of therapeutic drugs for tumors, autoimmune diseases, inflammatory diseases, or metabolic diseases.

8. The application of claim 6, wherein the autoimmune diseases include systemic lupus erythematosus, rheumatoid arthritis, systemic vasculitis, scleroderma, dermatomyositis, mixed connective tissue disease, autoimmune hemolytic anemia, thyroid autoimmune disease, ulcerative colitis, acute glomerulonephritis, rheumatic heart disease, and diabetes.

9. A method for coupling the oligodeoxynucleotide of any one of claim 1-3 to B lymphocyte, wherein the method comprises the following steps:
(1)modify the 3 'end of the oligodeoxynucleotides of any one of claim 1-3 to DBCO and the 5' end to Biotin, respectively;
(2) add excess of GDP-Azido-fucose to the solution of modified oligodeoxynucleotides obtained in step (1) and incubate to obtain GDP-fucose-oligodeoxynucleotides;
(3) add the GDP-fucose-oligodeoxynucleotide obtained in step (2) and α-1,3-fucosyltransferase to the B lymphocyte suspension and incubate to obtain B lymphocyte conjugated with oligodeoxynucleotides.

10. The method of claim 9, wherein in step (3), 100 µg/mL of α -1,3-fucosyltransferase and 10 µ M GDP fucose-oligodeoxynucleotide prepared in step (2) are added to 5 × 10⁸/mL B cells, respectively.
